# EUROPEAN PATENT APPLICATION

(11) **EP 1 920 776 A1**
(43) Date of publication of application: **14.05.2008**
(21) Application number: 07015969.4
(22) Date of filing: 14.08.2007
(51) Int. Cl.: A61K 36/06

(54) **Prevention of damage in hippocampus of fetus/newborn infant due to maternal gestational hypertension**

(30) Priority: 14.08.2006 US 503172
(71) Applicant: Kindway International Ltd., Causeway Bay Hong Kong (CN)
(72) Inventor: Chung, Chee-Keung, Kwun Town, Kowloon (HK); Zeng, Yuan-Shan, Zhi Xin Nan Lu, 510080 Guangzhou (CN)
(74) Representative: Oser, Andreas

(57) **Abstract**

The present invention provides a method for preventing or ameliorating neural damage in a mammalian fetus or a newborn infant, which is caused by maternal gestational hypertension (GH). The method includes orally administering germination activated sporoderm-broken *Ganoderma lucidum* spores ("GLSs") to a pregnant mammal having GH. The neural damage includes a decrease in hippocampal cell proliferation and neuron number. The present invention further provides a method for preventing or ameliorating the increased expression of hippocampal hypoxia inducible factor (HIF-1a) and vascular endothelial growth factor (VEGF) in a mammalian embryo caused by maternal GH.

## Description

### FIELD OF THE INVENTION

The present invention relates to a method for preventing or ameliorating neural damage in a mammalian fetus or newborn infant due to maternal gestational hypertension (GH). The method includes orally administering germination activated sporoderm-broken Ganoderma lucidum spores ("GLSs") to a pregnant mammal with GH. The neuronal damage includes a decrease in hippocampal cell proliferation and neuron number. The present invention further relates to a method for preventing or ameliorating the increased expression of hippocampal hypoxia inducible factor (HIF-1a) and vascular endothelial growth factor (VEGF) in a mammalian embryo caused by maternal GH.

### BACKGROUND OF THE INVENTION

Gestational hypertension (GH) is high blood pressure that develops after the twentieth week of pregnancy and returns to normal after delivery in women with previously normal blood pressure. A more serious condition, termed preeclampsia, is GH accompanied with protein in the urine. Preeclampsia can evolve into eclampsia, leading to maternal seizures. These pregnancy-related hypertension disorders may cause serious damage to the various organs of the pregnant woman and the fetus. Maternal complications can include pulmonary edema, thrombotic complications, renal failure, and death. In the United States, hypertensive disorders of pregnancy account for nearly 15 percent of maternal mortality. Long-term sequelae may also result. While women with chronic hypertension have an obvious long-term risk from the persistent hypertension, however, women with preeclampsia, despite the resolution of the disorder postpartum, are also at increased risk of cardiovascular disease in later life compared to women with pregnancies without preeclampsia

Fetal complications due to GH include growth restriction, prematurity, and stillbirth. GH may also cause developmental delays in the fetal organs in utero, postnatal declines in intelligence and many neuropsychological disorders. There is also evidence that the intrauterine milieu in a hypertensive pregnancy may, by mechanisms related to the failure of the fetus to exercise full growth potential, confer increased risk of cardiovascular events in adult life.

Ganoderma belongs to the Polyporaceae group of the Fungi family. Ganoderma is widely used in traditional Chinese medicine as an auxiliary treatment for a variety of medical conditions, such as hepatis, AIDS, cancer, and autoimmune diseases. The GLSs are the essence of Ganoderma and contain active ingredients such as polysaccharides, sterols, oleic acid, linoleic acid, triterpenes, ceramides and certain organic ions, all of which possessing strong bioactivity. The GLSs have also been used for the treatment of neurological disorders. To date, however, there have been no reports on the effects of GLSs on GH-related fetal damage.

### SUMMARY OF THE INVENTION

The present invention provides a method for preventing or ameliorating neural damage in a mammalian fetus or newborn offspring due to maternal gestational hypertension (GH). The method comprises administering to a pregnant female with GH an effective amount of germination activated sporoderm-broken *Ganoderma lucidum* spores (GLSs).

GLSs are prepared by (1) soaking Ganoderma spores in a solution which is water, saline, a nutritional solution, or a combination thereof to cause the spores to germinate; (2) placing the germination-treated Ganoderma spores in a culture box at a relative humidity of 65-98% and temperature of 18-48°C to cause the germinated Ganoderma spores to activate; and (3) breaking the sporoderm of the germination activated Ganoderma spores to produce the sporoderm-broken GLSs.

The pregnant female is suffered from gestational hypertension.

The pregnant female is preferred to be a human. Alternatively, the pregnant female is a rodent.

The GH is preferred to be preeclampsia. Possibly, in an animal model, the preeclampsia is induced by N-omega-nitro-L-arginine methyl ester. The induced preeclampsia demonstrates similar symptoms of preeclampsia as in humans.

One form of the neural damage is a decrease in hippocampal cell proliferation.

Another form of neural damage is a decrease in neuron number. The neural damage occurs in the hippocampus region of the brain.

The GLSs is preferred to be administered orally.

The effective amount of GLSs is preferred to be between 0.01 and 20 g / kg body weight / day, and more favorably, between 0.1 and 20 g / kg body weight / day.

The present invention further provides a method for preventing or ameliorating the increased expression of hippocampal hypoxia inducible factor (HIF-1a) and vascular endothelial growth factor (VEGF) in a mammalian embryo caused by maternal GH. The method comprises administering to a pregnant female with GH an effective amount of germination activated sporoderm-broken *Ganoderma lucidum* spores (GLSs).

### BRIEF DESCRIPTION OF DRAWINGS

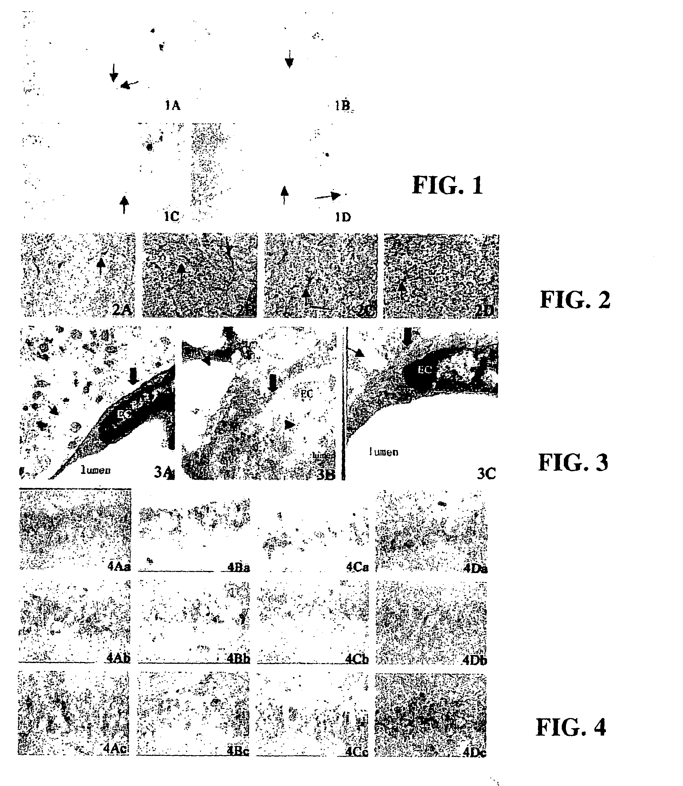

Figure 1 is a composite of pictures showing immunohistochemical staining of BrdU positive nuclei (↑) at subgranular zone (SGZ) of the hippocampus in 30-old (P30) rats born from the experimental pregnant rats at 50× magnification (insert at upper right corner of each panel is 200×). Panel 1A: normal control group; panel 1B: L-NAME + distilled water group; panel 1C: L-NAME + arginine group; panel 1D: L-NAME + GLSs group.
Figure 2 is a composite of pictures showing the microvessel (↑) at the hippocampus in 21-day old (E21) embryos, using vWF immunohistochemical staining 100× magnification. Panel 2A: normal control group; panel 2B: L-NAME + distilled water group; panel 2C: L-NAME + arginine group; panel 2D: L-NAME + GLSs group.
Figure 3 is a composite of pictures showing the ultrastructure (↑ indicates the peripheral vascular structure and 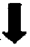 indicates the vascular basement membrane) of blood capillaries at the CA1 area of the hippocampus in P30 rats. EC is the vascular endothelial cell; lumen indicates lumen in the blood vessel. Transmission electron microscope 12000×. Panel 3A: normal control group; panel 3B: L-NAME + distilled water group; panel 3C: L-NAME + Ganoderma spore group. The capillary basement membrane becomes thicker (panel 3B, 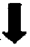 ), the peripheral structure appears vacuolar (panel 3B, ►) and the endothelial cells that emerge are also vacuolar (panel 3B, T) in the L-NAME + distilled water group.
Figure 4 is a composite of pictures showing the cell layer and the neuron body at the CA1, CA3 and DG areas of the hippocampus in P30 rats. Neutral red staining, 200x. Panel 1A: normal control group; panel 1B: L-NAME + distilled water group; panel 1C: L-NAME + arginine group; panel 1D: L-NAME + GLSs group; a: CA1 area; b: CA3 area; c: DG area. DG: Dentate gyrus. CA1-CA3: the *Cornu Ammonis* fields. Although there is a lack of consensus relating to terms describing the hippocampus and the adjacent cortex, the term "hippocampal formation" generally applies to the dentate gyrus, the *Cornu Ammonis* fields CA1-CA3 (and CA4, frequently called the hilus and considered part of the dentate gyrus), and the subiculum. The CA1, CA2 and CA3 fields make up the hippocampus proper.

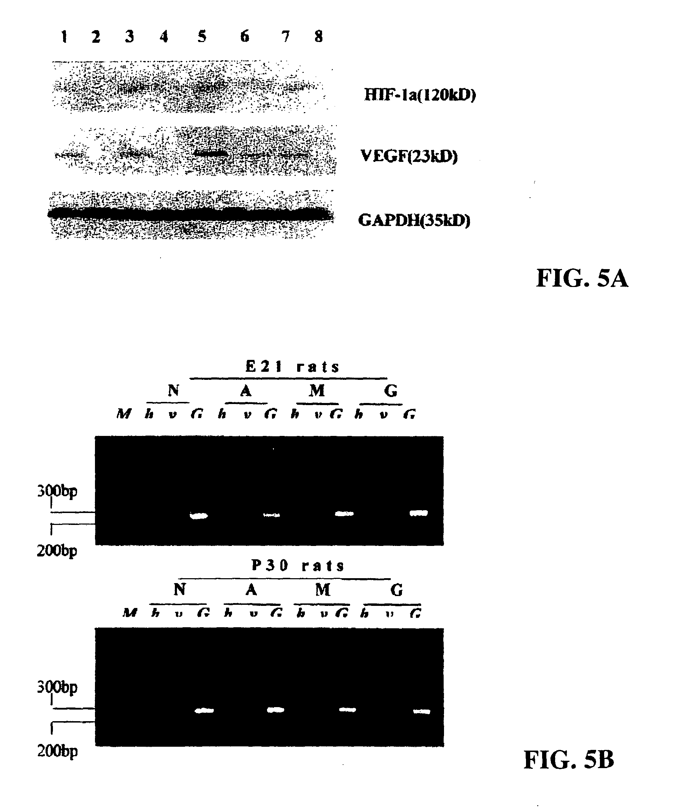

Figure 5A is a picture of a Western Blot showing HIF-1a and VEGF expression in E21 and P30 hippocampal tissue. N- Normal control group; M- L-NAME + distilled water group; A-L-NAME + arginine group; G-L-NAME + GLSs.
Figure 5B is a composite of pictures showing RT-PCR results of HIF-1a and VEGF expression in E21 and P30 hippocampal tissue. N- Normal control group; M-L-NAME + distilled water group; A-L-NAME + arginine group; G-L-NAME + GLSs.

### DETAILED DESCRIPTION OF THE INVENTION

This invention focuses on the investigation of neural damage and increased expression of hypoxia inducible factor (HIF-1a) and vascular endothelial growth factor (VEGF) in hippocampus of a mammalian fetus and/or newborn infant due to maternal gestational hypertension (GH) and the effects of germination activated sporoderm-broken Ganoderma lucidum spore powders (GLSs) on prevention or amelioration of such damage. To facilitate the research on neural damage in the mammalian fetus/newborn infant due to maternal GH, an animal model of preeclampsia is employed according to Yallampalli C. et al., Am J. Obstet Gynecol., 169(5):1316-1320 (1993) and Helmbrecht GD et al., Am. J. Obstet. Gynecol., 175(4):800-805 (1996). The animal model involves the use of a non-selective inhibitor of nitric oxide synthase (NOS), N-omega-nitro-L-arginine-methyl ester (L-NAME), to induce a preeclampsia-like syndrome of hypertension, proteinuria, intrauterine growth restriction and renal glomerular capillary endothelial lesions in pregnant rats. This model is well-recognized in the obstetrical field to produce symptons similar to those of preeclampsia in pregnant humans.

The theory behind the use of L-NAME to induce a preeclampsia-like syndrome is based on the observation that in pregnant women with GH, the level of nitric oxide (NO) in the blood is significantly lower than that in normal pregnant women. (Wang Y., et al., Am J Obstet Gynecol., 190(3):817-824 (2004)). NO is produced by endothelial cells and is involved in the regulation of vascular tone, platelet aggregation, neurotransmission and immune activation. NO, formerly known as EDRF (endothelin-derived relaxing factor), however, is synthesized by the oxidative deamination of a guanidino nitrogen of L-arginine by a flavin-containing enzyme, nitric oxide synthase (NOS). In other words, L-arginine is the substrate for NOS to produce NO. Therefore, a replacement of L-arginine with L-NAME, which is an analog of L-arginine, blocks the synthesis of NO and in turn reduces the production of NO in the pregnant rodents, which mimics the reduction of NO in pregnant humans.

As will be shown in the following experimental design and results, *infra,* the inventors of the present invention discovered that the maternal GH caused decrease in cell proliferation and neuronal number at the hippocampus region of the brain in fetuses and newborn infants. An increase in expression of hippocampal hypoxia inducible factor (HIF-1a) and vascular endothelial growth factor (VEGF) in a mammalian embryo was also discovered.

The hippocampus is located in the medial temporal lobe of the brain. It forms a part of the limbic system and plays a part in memory and spatial navigation. The name "hippocampus" derives from its curved shape in coronal sections of the brain, which resembles a seahorse (Greek: *hippokampos).* In Alzheimer's disease, the hippocampus becomes one of the first regions of the brain to suffer damage. The damage in hippocampus includes, but is not limited to, memory problems and disorientation.

Thus, a damage at the hippocampus of the brain in fetuses and newborn infants can lead to damage to the fetuses' and newborn infants' memory loss and long-term learning ability.

In describing preferred embodiments of the present invention, specific terminology is employed for the sake of clarity. However, the invention is not intended to be limited to the specific terminology so selected. It is to be understood that each specific element includes all technical equivalents which operate in a similar manner to accomplish a similar purpose.

One aspect of the present invention relates to a method for preventing or ameliorating neural damage to the fetus/newborn infant due to maternal GH. The method comprises the step of administering to a pregnant female with GH an effective amount of GLSs.

The pregnant female can be any mammal, including but not limited to, human, pets such as dogs and cats, farm animals such as sheep, goat, cow, and pig, and laboratory animals such as mouse, rat, guinea pig, rabbit, monkey and baboon.

GLSs is a brown powder that is slightly soluble in water. Bioactive GLSs can be produced in a process containing the following steps:
*I. Induction of germination:* Mature and perfect spores of Ganoderma lucidum are carefully selected to undergo a soaking process to induce germination. Spores are kept in clear or distilled water, biological saline solution, or other nutritional solutions that could enable the spores of Ganoderma lucidum to germinate rapidly. Examples of nutritional solutions include coconut juice or a 1-5% malt extract solution, 0.5-25% extracts of Ganoderma lucidum sporocarps or Ganoderma lucidum capillitia, 0.1-5% of culture solution containing biotin, 0.1-3% of culture solution containing potassium phosphate (monobasic) and magnesium sulfate. The choice of solution would depend on the soaking time required, the amount of spores to be processed and other such factors as availability of materials. One or more of the above germination solutions could be used, with the amount added being 0.1 -5 times the weight of the spores of Ganoderma lucidum. The soaking time can be determined according to the temperature of the water, and usually the soaking was carried out for 30 min to 8 hours with the temperature of the water at 20-43°C. Preferably, the soaking time is 2-4 hours, and the temperature of water is 25-35°C.
*II. Activation culture:* The spores of Ganoderma lucidum are removed from the soaking solution and excess solution is eliminated by allowing it to drip. The spores are then placed in a well-ventilated culturing box at a constant temperature and humidity so that spore culture activation could be carried out. The relative humidity of the culture was generally set at 65-98%, the culture temperature set at 18-48°C. and the activation time lasted from 30 min to 24 hours. Preferably humidity is 85-97% and temperature is 25-35°C. During activation, the cell walls of the spores of Ganoderma lucidum are clearly softened such that it was easier to penetrate the cell walls of the spores. The activation of spores of Ganoderma lucidum typically reaches a rate of more than 95%.
*III. Treatment of the epispores:* After the germination/activation process, the spores are treated by enzymolysis. This process is carried out at a low temperature and under conditions such that enzyme activity is maintained, using chitinase, cellulase, or other enzymes, which are commonly used in the industry. The process is complete when the epispores lost their resilience and became brittle. Alternatively, physical treatments are carried out to penetrate the cell walls, for example, micronization, roll pressing, grinding, super high pressure microstream treatment, and other mechanical methods commonly used in the industry could be carried out, with a penetration rate of over 99%.
*IV. Drying or extraction:* Drying is carried out at low temperature using standard methods including freeze-drying or vacuum-drying etc., which are commonly used in the industry. The obtained product has a moisture content less than 4%. After drying, the bioactive substances are extracted by water or alcohol, or by thin film condensation. The extracted bioactive substances can be further purified by dialysis to ensure no contamination in the final products. The final product can be made into purified powders, extract pastes, solutions for injection, or for oral consumption.

A more detailed description for production of GLSs can be found in U.S. Patent No. 6,316,002, which is hereby incorporated by reference. GLSs is commercially available from Kindway International Ltd./Holistol International Ltd., Sweden and Hong Kong.

The effective amount of GLSs is a dosage which is useful for preventing or ameliorating GH-related neural damage in a fetus/newborn infant. Toxicity and therapeutic efficacy of GLSs can be determined by standard pharmaceutical procedures in cell culture or experimental animal models, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. The data obtained from cell culture assays and animal studies can be used in formulating a range of dosages for use in humans. Generally, appropriate dosages for administering GLSs may range, for example, from about 0.01 g / kg body weight / day to about 20 g / kg body weight / day. In one embodiment, the effective amount of GLSs is between 0.1 and 20 g / kg body weight / day. In another embodiment, the effective amount of GLSs is between 1 and 10 g / kg body weight / day. In yet another embodiment, the effective amount of GLSS is between 8 g / kg body weight / day.

GLSs are preferred to be administered orally. The administration can be in one dose, or at intervals such as three times daily, twice daily, once daily, once every other day, or once weekly. Dosage schedules for administration of GLSs can be adjusted based on the individual conditions and needs of the target. Continuous infusions may also be used after the bolus dose. The effects of any particular dosage can be monitored by suitable bioassays.

GLSs can also be formulated into a pharmaceutical composition with a pharmaceutically acceptable carrier. As used herein the language "pharmaceutically acceptable carrier" is intended to include any and all solvents, solubilizers, fillers, stabilizers, binders, absorbents, bases, buffering agents, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary agents can also be incorporated into the compositions.

The pharmaceutical composition of the present invention is formulated to be compatible with its intended route of administration, e.g., oral or parenteral administration. Oral compositions generally include an inert diluent or an edible carrier. They can be enclosed in gelatin capsules or compressed into tablets. For the purpose of oral therapeutic administration, the active compound can be incorporated with a solid carrier and used in the form of tablets, troches, or capsules. Oral compositions can also be prepared using a fluid carrier. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, such as sodium chloride, sugars, polyalcohols (*e.g.*, manitol, sorbitol, *etc.*) in the composition. Prolonged absorption of the injectable compositions can be brought about by including in the composition an agent which delays absorption, for example, aluminum monostearate and gelatin.

Sterile injectable solutions can be prepared by incorporating the GLSs in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

It is advantageous to formulate oral or parenteral compositions in dosage unit form for ease of administration and uniformity of dosage. Dosage unit form as used herein includes physically discrete units suited as unitary dosages for the subject to be treated; each unit contains a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals. The pharmaceutical compositions can be included in a container, pack, or dispenser together with instructions for administration.

The following examples are illustrative, but not limiting the scope of the present invention. Reasonable variations, such as those occur to reasonable artisan, can be made herein without departing from the scope of the present invention. Also, in describing the invention, specific terminology is employed for the sake of clarity. However, the invention is not intended to be limited to the specific terminology so selected. It is to be understood that each specific element includes all technical equivalents which operate in a similar manner to accomplish a similar purpose.

### EXPERIMENTAL DESIGN

### I. Materials and methods

*Animal groups and administration of medication.* Gestational hypertension (GH) was produced in rats using the L-NAME (a type of NOS inhibitor) method of Yallampalli et al. [Yallampalli et al., Am J Obstet Gynecol., 1993, 169:1316-1320]. Forty mature (11-12 weeks old) SD female rats weighing 200-230 g; 10 mature (13-15 weeks old) SD male rats weighing 250-300 g. At a ratio of 1:2 female:male, the rats were paired in cages, on the second day the female rats' vaginal secretions were observed (smear technique) and those containing sperm were considered to be at day 0 of pregnancy (E0). These pregnant rats were divided into (1) normal control group - injected i.p. with normal saline daily + gastric feeding of distilled water daily, from 14 to 20 days of pregnancy (E14-20); (2) L-NAME + distilled water group (injected i.p. with L-NAME + gastric feeding of distilled water); (3) L-NAME + arginine group (injected i.p. with L-NAME + gastric feeding of L-arginine 32mg/kg/day) and (4) L-NAME + GLSs group (injected i.p. with L-NAME + gastric feeding of GLSs 8g/kg/day). L-NAME was given by two injections at a total dose of 60 mg/kg/day. Two hours after each injection, the pregnant rats received gastric feeding of distilled water, L-arginine or GLSs. The GLSs were provided by Kindway International Ltd./Holistol International Ltd., license number GANSPSP04040104. The L-arginine was purchased from the Amresco Company.

*Preparation of histological materials.* Some of the pregnant rats were anesthetized at E21 day with an i.p. injection of 1% pentobarbital (35 mg/kg). The embryos were removed from the uterus. Some were placed in a freezer dish and the hippocampal tissue was removed from the brains of the embryos, placed in an Eppendorf tube and stored at -80°C to await testing. The rest of the embryonic brains were fixed in a 4% paraformaldehyde solution. Another group of pregnant rats went into normal labor, and the young rats were fed and raised until 30 days of pregnancy (P30). Some of the young rats received the preceding pentobarbital anesthesia and their hippocampal tissue was placed in a freezer dish and stored to await testing. The rest of the rats in the P30 group received pentobarbital anesthesia as above, their chests were opened and an aortic cannula inserted through the left ventricle. One group of young rats was first quickly perfused with normal saline and then 4% paraformaldehyde 0.1 mol/L phosphate buffer (PB) (pH 7.4). Then the fixed cerebral corona was continuously frozen and sectioned at a thickness of 15 µm. Sectioning started at the alveus of the hippocampus, one section every 300 µm, and six sections were taken from each brain, for a collection of six sets of sections.

Another group of animals was quickly perfused with prechilled normal saline, and follow with 0.1 mol/L PB (pH 7.4) containing 2% glutaraldehyde + 2% paraformaldehyde. The brain was extracted, fixed and placed at 4°C overnight. The brain sample was sectioned in a vibrating microtome to a thickness of 100 µm, fixed in 1% osmic acid, dehydrated, embedded in resin and semi-thin sectioning performed. After confirming that it was the CA1 area, ultra-thin sectioning was performed, then uranyl acetate and lead citrate double electron staining, then transmission electron microscopy for observation and photography.

### II. Flow Cytometry

Hippocampal tissue separated from E21 day rat embryos was placed in a culture dish and sheared into pieces, 0.01 mol/L phosphate buffered saline (PBS) was added and repeatedly triturated, filtered through a 100-mesh screen, and the single-cell suspension obtained was fixed in 75% alcohol at 4°C to await testing. Before testing, the extracted cell suspension was centrifuged to remove the supernatant, 100 µL RNase (0.1 mg/L) added, placed at 37°C for 30 min, after rinsing with normal saline, propidium iodine (PI) stain (0.05 mg/L, 0.03% Triton X-100 0.5 mL) was added, placed at 4°C protected from light to react for 30 min, then measured. In flow cytometry, a 488 nm laser used Cell Fit software to collect 10,000 cells/specimen cell cycle data was analyzed and one-way ANOVA testing performed for statistical processing.

### III. Immunohistochemical staining

The hippocampal tissue sections were fixed with H₂O₂ and normal goat serum. The first antibody incubation solution was added dropwise and placed at 4°C overnight. The first antibody types and concentrations included: rabbit antibody hypoxia inducible factor-1a (HIF-1a) (Boshide, 1:200), rat antibody vascular endothelial growth factor (VEGF) (Santa Cruz, 1:200), rat antibody factor eight related antigen (vWF [von Willebrand's factor]) (Beijing Zhongshan, working solution). After dropwise PBS washing and after adding the second antibody and performing specific antigen binding capacity (SABC) incubation, DAB was used for development, then hematoxylin counterstaining, dehydration, clarification and sealing were performed.

*BrdU labeling.* Five animals from the various P30 groups of rats were selected at random, received abdominal cavity injections, and a thymine analogue, 5-bromodeoxyuridine (BrdU) (Sigma) label, was used to locate proliferating cells. The BrdU was dissolved in normal saline containing 0.007N NaOH and was freshly prepared before use. 50 mg/kg was injected into the abdominal cavity and, two hours before perfusion, the rats received one injection. BrdU immunohistochemical staining was performed with reference to the methods of Kuhn et al. [Kuhn et al., J. Neurosci, 1996, 16:2027-2033]. Briefly, a tissue section was washed with 0.01 mol/L PBS three times (5 min for each wash); incubated in 3% H₂O₂ at room temperature for 30 min; washed with 0.01 mol/L PBS three times ((5 min for each wash); incubated with 50% formamide/2X SSC at 65°C incubator for 2 h; washed with 2X SSC three times (5 min for.each wash); incubated with 2N HCl at 37°C for 30 min; washed with 0.1 mol/L boricate buffer (pH 8.0) three times (5 min for each wash); washed with 0.01 mol/L PBS three times (5 min for each wash); blocked with 1:50 normal goat serum for 30 min; incubated withmouse antibody BrdU monoclonal antibody (Sigma, 1:400) at 4°C overnight; washed in 0.01 mol/L PBS three times (5 min for each wash); reacted with 1:200 biotin-labeled sheep anti-mouse IgG at room temperature for 30 min; washed with 0.01 mol/L PBS three times (5 min for each wash); incubated with 1:100 SABC at room temperature for 30 min; developed color with DAB; counterstained with hematoxylin, and then subjected to conventional fixation and dehydration.

*Morphometric analysis.* Various cell layer thicknesses from various areas of the hippocampus were measured and a neuron body count obtained. Under a low-magnification microscope, two visual fields were randomly selected from the CA1, CA3 and DG regions. Under a high-magnification microscope (200×), the cell layer thickness was measured and the number of nucleated neuron bodies counted. The cell layer thicknesses or body numbers are the averages of two visual fields.

*Hippocampal microvessel density:* The Weidner counting method was used to determine the microvessel standard [Weidner et al., Am J Pathol. 1995, 147:9-19], which uses the positive vWF staining of cytoplasm as indication of vascular endothelial cells. The examined areas were microvessels in the stratum pyramide and stratum molecular layers. Under a light microscope (200×), microvessel numbers were counted in five visual fields. The microvessel density was the average of the five fields.

*Hippocampus BrdU positive cell counts.* For each animal, the BrdU positive cell numbers were counted in six brain sections from the dentate gyrus granulocyte layer. According to the methods of Kuhn et. al [Kuhn et al., J Neurosci. 1996, 16:2027-2033], the subgranular zone (SGZ) was defined as an area two granule cells wide within each granular layer. One side of the hippocampal SGZ from each of the six brain sections was used to obtain total BrdU positive cell counts.

### IV. Reverse Transcription-Polymerase Chain Reaction

For each group, three examples of separated hippocampal tissue were collected and total tissue RNA was extracted according to the Trizol reagent instructions. Five µg of RNA underwent the RT reaction under the following conditions cDNA was synthesized: 70°C for 5 min, 42°C for 60 min, 94°C for 5 min. The HIF-1a primer sequence was: upstream primer: 5' GGC TGG GCA ACT ACG TCA TC 3' (SEQ ID NO:1) downstream primer: 5' CCA TGC ACC TTA ACA TCC CA 3' (SEQ ID NO:2); the VEGF primer sequence was: upstream primer: 5' AAT TGA GAC CCT GGT GGA CAT C 3' (SEQ ID NO:3); downstream primer: 5' TCT TTC TTT GGT CTG CAT TCA C 3' (SEQ ID NO:4); the internal reference GAPDH primer sequence was: upstream primer: 5' GTG CTG AGT ATG TCG TGG AGT 3' (SEQ ID NO:5); downstream primer: 5' GAT GGC ATG GAC TGT GGT CA 3' (SEQ ID NO:6). The PCR reaction was performed according to the following sequence: predenaturation at 94°C for 2.5 min, then the following for 30 cycles: denaturation at 94°C for 45 sec, renaturation at 60°C for 1 min; extension at 72°C for 1.5 sec; when the cycles are complete, extension at 72°C for 5 min then stop the reaction. The PCR product is then separated by 2% agarose gel electrophoresis.

### V. Western Blot

Three examples of separated hippocampal tissue were collected from each group for homogenization. 20 µg of protein was separated by 10% SDS-PAGE and electronically transferred onto PVDF membrane. The membrane was blocked with 5% non-fat milk powder at room temperature for 2 h, then incubated with a mouse anti-VEGF monoclonal antibody (Santa Cruz 1:200)or a rabbit anti-HIF-1a polyclonal antibody (Wuhan Boshide, 1:200) at 4°C overnight. The membranes were washed with phosphate-buffered saline and Tween (PBST), and incubated with horseradish peroxidase conjugated goat anti-mouse IgG (Santa Cruz, 1:5000, for mouse anti-VEGF antibody) or goat anti-rabbit IgG (Santa Cruz, 1:5000, for rabbit anti-HIF-1a antibody) at room temperature for 2 h. A horseradish peroxidase labeled mouse anti-GAPDH monoclonal antibody (Shanghai Kangcheng, 1:10000) was added dropwise with the horseradish peroxidase conjugated goat anti-mouse IgG or goat anti-rabbit IgG. The membrane was then washed with PBST and developed using the ECL [enhanced chemiluminescense] chemical photogenesis technique.

### VI. Statistical analysis

All experimental data underwent statistical processing using single-factor variance analysis with the aid of the SPSS 10.0 statistical software and calculated results were expressed as the mean ± standard deviation.

### VII. Results

*Flow cytometry results.* Table 1 is a comparison of the four groups of rat embryo brain hippocampal cell cycles at E21 day. The results show that application of L-NAME significantly reduces the percentage of hippocampal cells in the DNA synthesis phase (S phase), and elevate the percentage G0/G1 phase cells. The number of S phase cell in the L-NAME + GS group, however, was statistically different from that of the L-NAME + DW group and was not statistically different from that of the normal control group. Although the animals in L-NAME + Arg group also showed an increase in S phase cells compared to the L-NAME + DW group, the increase was not statistically significant. The L-NAME + Arg group, however, has the highest percentage of G1/G0 phase cells, which is statistically significant compared to those of the normal control group and the L-NAME + Ganoderma spore group. The results show that the use of GLSs restores the number of S phase hippocampal cell in E21 day rat embryo to near normal levels.

**Table 1. Percentages (x ± sx, %) of E21 Day Rat Embryo Hippocampal Cell at Various Phases of Cell Cycle**

| Groups | S | G1/G0 | G2/M |
|---|---|---|---|
| Normal control | 18.30±3.18^{a} | 80.06±2.69^{b} | 1.66±0.56^{c} |
| L-NAME+DW | 8.16±0.58^{d} | 88.82±0.62^{e} | 3.04±0.98^{f} |
| L-NAME+L-arg | 15.34±2.92^{g} | 82.74±2.75^{h} | 1.44±0.44ⁱ |
| L-NAME+GS | 16.18±2.81^{j} | 81.76±2.05^{k} | 1.74±0.76^{l} |

| | | | |
|---|---|---|---|
| DW: distilled water, L-arg: L-arginine; GS: Ganoderma spore Single factor variance analysis (One way ANOVA): S phase: a*VSd,* a*VS*j*,* d*VS*j *P*<0.05; a*VS*g*,* d*VS*g *P* >0.05 G1/G0 phase: b*VS*e*,* e*VS*k *P* <0.05; b*VS*h, b*VS*k, h*VS*k *P* >0.05 G2/M phase: c*VS*f*,* c*VS*i*,* c*VS*l*,* f*VS*i, f*VS*l, i*VS*l *P* >0.05 | | | |

*BrdU labeling.* As shown in Figure 1, BrdU positive cells were mainly distributed in the hippocampus dentate gyrus of P30 young rats. Table 2 shows that the L-NAME + DW group and the L-NAME + L-arg group had fewer BrdU positive cells than the normal control group (see also Figures 1A-1C). Compared to the L-NAME + DW group, the L-NAME + GLSs group had a increased number BrdU positive cells (Figure 1D) that is not significantly different from that of the normal control group. This result indicates that GLSs are able to restore BrdU positive cell counts to normal levels.

*Hippocampus microvessel density and ultrastructure changes.* The results in Table 3 indicate that in the L-NAME + DW group, E21 day hippocampus microvessel density was clearly increased (Figure 2B). After the use of GLSs, microvessel density (Figure 2D, Table 4) was reduced to the levels of the normal control group (Figure 2A). Although microvessel density decreased with the administration of arginine (Figure 2C, Table 4), the amount of the reduction was not as great as that with the use of GLSs. P30 day hippocampus microvessel density was not statistically different among the four groups of young rats. However, compared to the normal control group, with the administration of L-NAME, vacuoles appeared in the vascular endothelial cells at the hippocampus CA1 area capillary wall ultrastructure (Figure 3A) (Figure 3B), the vessels' peritubular structure was cloudy and unclear and also had vacuoles and the vessels' basement membrane was thicker. However, after the use of GLSs, CA1 area vascular endothelial cells and vascular wall structures were more complete (Figure 3C).

**Table 3. BrdU Positive Cell Counts (x ± sd) at Hippocampus Dentate Gyrus in P30 Rats.**

| Groups | BrdU positive cells |
|---|---|
| Normal control | 50.00±4.63^{a} |
| L-NAME+DW | 32.60±3.37^{b} |
| L-NAME+L-arg | 38.00±1.04^{c} |
| L-NAME+GS | 43.00±2.70^{d} |

| | |
|---|---|
| DW: distilled water, L-arg: L-arginine; GS: Ganoderma spore Single factor variance analysis (One-way ANOVA): a*VS*b*,* a*VS*c *P*<0.01; b*VS*d*, P*< 0.05; a*VS*c, a*VS*d*,* bV*S*c*,* c*VS*d *P*> 0.05 | |

**Table 4. Microvessel Density (x ± sd) at the Hippocampus in E21 and P30 Rats.**

| Groups | E21 | P30 |
|---|---|---|
| Normal control | 23.69±2.33^{a} | 71.33±8.08^{b} |
| L-NAME+DW | 54.30±6.54^{c} | 77.33±3.78^{d} |
| L-NAME+L-arg | 39.80±4.79^{e} | 75.25±3.86^{f} |
| L-NAME+GS | 27.54±4.13^{g} | 68.67±8.50^{h} |

| | | |
|---|---|---|
| DW: distilled water, L-arg: L-arginine; GS: Ganoderma spore Single factor variance analysis (One-way ANOVA).: E21 □a*VS*c, c*VS*g *P*<0.01; a*VS*e, c*VS*e*,* e*VS*g *P*<0.05; aVSg *P*>0.05 P30 □ b*VS*d, b*VS*f, b*VS*h*,* d*VS*f*,* d*VS*h*,* f*VS*h *P> 0.05* | | |

*Hippocampal cell layer thickness measurement and neuron body count results.* After neutral red staining, the L-NAME + DW group (Figures 4, panels Ba, Bb, and Bc) and the L-NAME + L-arg group (Figures 4, panels Ca, Cb, and Cc) P30 rats in the hippocampus CA1 area, CA3 area and DG cell layer thickness were observed and compared to the normal control group (Figures 4, panels Aa, Ab, and Ac). The neuron body count was clearly reduced (Table 5). After the use of GLSs, CA1 area, CA3 area and DG cell layer thickness and neuron body counts all achieved restorative increases (Figures 4, panels Da, Db, and Dc, Table 5). The results show that, after the use of GLSs, it is possible to achieve a restorative increase in the hippocampus neuron body counts, and this effect was not observed with the administration of arginine.

**Table 5. Hippocampal cell Layer Thickness (x ± sd, µm) and Neuron Body Counts (x ± sd, num) in P30 Rats.**

| Groups | CA1(µm) | CA1(num) | CA3(µm) | CA3(num) | DG(µm) |
|---|---|---|---|---|---|
| Normal control | 130.01±8.03^{a} | 41.20±1.35^{b} | 104.42±7.21^{c} | 38.00±3.01^{d} | 84.06±1.37^{e} |
| L-NAME+DW | 100.78±3.65^{g} | 27.60±1.36^{h} | 79.30±6.69ⁱ | 26.60±1.36^{j} | 59.41±5.31^{k} |
| L-NAME+L-arg | 115.23±9.49^{m} | 31.80±1.15ⁿ | 90.64±6.18° | 23.00±0.83^{p} | 64.87±4.42^{q} |
| L-NAME+GS | 125.60±7.30^{s} | 38.00±1.58^{t} | 93.70±9.78^{u} | 35.20±1.28^{v} | 75.12±3.81^{w} |

| | | | | | |
|---|---|---|---|---|---|
| Single factor variance analysis (One-way ANOVA): CA1(µm): a*VS*g, g*V*Ss *P*<0.05; a*VS*m, a*VS*s, g*VS*m, m*VS*s *P*>0.05 CA1(num): b*VS*h*,* b*VS*n*,* b*VS*h, n*VS*t, h*VS*n *P*<0.05; b*VS*t*,* n*VS*t *P*>0.05 CA3(µm): c*VS*i *P*<0.05; c*VS*o, c*VS*u, i*VS*o, i*VS*u, o*VS*u*, P*>0.05 CA3(num): d*VS*j*,* d*VS*p, p*VS*v *P*<0.01; d*VS*v*;* j*VS*p *P*>0.05 DG(µm): e*VS*k, e*VS*q*,* k*VS*w*,* e*VS*w, q*VS*w *P*<0.05; k*VS*q *P*>0.05 | | | | | |

*Hippocampus HIF-1a and VEGF expression.* RT-PCR and Western-Blot tests were performed on HIF-1a and VEGF mRNA and protein expression levels in E21 and P30 hippocampal tissue. The test results are shown in Figures 5A and 5B. In terms of HIF-1a mRNA and its protein expression in the E21 hippocampus for the various groups, in the L-NAME + distilled water group and the L-NAME + arginine group HIF-1a mRNA and its protein expression levels should be higher than in the normal control group (Figures 5A and 5B). After the use of GLSs, HIF-1a mRNA and its protein expression showed no clear increase. In the normal control group P30 hippocampus, HIF-1a mRNA and its protein expression were not detected. Then the L-NAME + distilled water group and the L-NAME + arginine group all had detectable HIF-1a mRNA and protein expression levels. After the use of GLSs, HIF-1a mRNA and its protein expression were not seen. At the same time, immunohistochemical testing of E21 and P30 hippocampal tissue HIF-1a protein expression in the various groups was consistent with the results for the Western-Blot test.

For the normal control group VEGF mRNA and its protein expression in the E21 hippocampus could be detected; however, VEGF mRNA and its protein expression could not be detected in the P30 hippocampus of the normal control group (Figures 5A and 5B). In the P30 hippocampus, in the L-NAME + distilled water group, VEGF mRNA and its protein expression could still be detected. After the use of Ganoderma spore, in the E21 hippocampus, VEGF mRNA and its protein expression could be detected, yet VEGF mRNA and its protein expression could not be detected in the P30 hippocampus. This situation is similar to that of VEGF mRNA and its protein expression in the normal control group. The immunohistochemical results for E21 and P30 in the hippocampal tissue of the various groups were similar to those for the Western-Blot test results above.

### VIII. Discussion

This study demonstrated that GH, especially preeclampsia, in pregnant female resulted in increased expression of hippocampal hypoxia inducible factor (HIF-1a) and vascular endothelial growth factor (VEGF) in embryos and the expression continued in newborn infant. The GLSs treatment restored the hippocampal HIF-1a and VEGF expression to normal levels, and prevented the decrease in hippocampal cell proliferation and neuron number and the development of abnormality in hippocampal tissue.

The embodiments illustrated and discussed in this specification are intended only to teach those skilled in the art the best way known to the inventors to make and use the invention. Nothing in this specification should be considered as limiting the scope of the present invention. The above-described embodiments of the invention may be modified or varied, and elements added or omitted, without departing from the invention, as appreciated by those skilled in the art in light of the above teachings. It is therefore to be understood that, within the scope of the claims and their equivalents, the invention may be practiced otherwise than as specifically described.

## Claims

1. Germination activated sporoderm-broken *Ganoderma lucidum* spores (GLSs) for preventing or ameliorating neural damage in a mammalian fetus or newborn offspring.

2. The *Ganoderma lucidum* spores (GLSs) according to claim 1, wherein the neural damage in a mammalian fetus or newborn offspring is due to maternal gestational hypertension, and wherein the GLSs is for administeration to a female being pregnant with said mammalian fetus wherein said pregnant female has gestational hypertension.

3. The *Ganoderma lucidum* spores (GLSs) according to claim 2, wherein said pregnant female is a human.

4. The *Ganoderma lucidum* spores (GLSs) according to claim 2, wherein said pregnant female is a rodent.

5. The *Ganoderma lucidum* spores (GLSs) according to anyone of claims 2 to 5, wherein said gestational hypertension is preeclampsia.

6. The *Ganoderma lucidum* spores (GLSs) according to claim 5, wherein said preeclampsia is caused by N-omega-nitro-L-arginine methyl ester.

7. The *Ganoderma lucidum* spores (GLSs) according to anyone of the preceding claims, wherein said neural damage is a decrease in cell proliferation.

8. The *Ganoderma lucidum* spores (GLSs) according to anyone of the preceding claims, wherein said neuronal damage is a decrease in neuron number.

9. The *Ganoderma lucidum* spores (GLSs) according to anyone of the preceding claims, wherein said GLSs are formulated to be administered orally.

10. The *Ganoderma lucidum* spores (GLSs) according to anyone of the preceding claims, wherein said neural damage is in a hippocampus region of a brain.

11. The *Ganoderma lucidum* spores (GLSs) according to anyone of the preceding claims, wherein said GLSs are prepared by soaking Ganoderma spores in a solution which is selected from the group consisting of water, saline, and a nutritional solution to cause the spores to germinate; placing said germination-treated Ganoderma spores in a culture box at a relative humidity of 65-98% and temperature of 18-48°C to cause the germinated Ganoderma spores to activate; and breaking sporoderm of said germination activated Ganoderma spores to produce said GLSs.

12. The *Ganoderma lucidum* spores (GLSs) according to anyone of the preceding claims, wherein said GLSs is for administration in an amount of between 0.01 and 20 g / kg body weight / day.

13. The *Ganoderma lucidum* spores (GLSs) according to claim 12, wherein said effective amount is between 0.1 and 20 g / kg body weight / day.

14. A use of germination activated sporoderm-broken *Ganoderma lucidum* spores (GLSs) for the manufacture of a medicament for preventing or ameliorating neural damage in a mammalian fetus or newborn offspring due to maternal gestational hypertension, wherein GLSs are administered to a female being pregnant with said mammalian fetus ; wherein said pregnant female has gestational hypertension.

15. Germination activated sporoderm-broken *Ganoderma lucidum* spores (GLSs) for preventing or ameliorating increased expression of hippocampal hypoxia inducible factor (HIF-1a) in a mammalian embryo.

16. The *Ganoderma lucidum* spores (GLSs) according to claim 15, wherein the increased expression of HIF-1a is due to maternal gestational hypertension, and wherein the GLSs is for administration to a female pregnant with said mammalian embryo; wherein said pregnant female has gestational hypertension.

17. The *Ganoderma lucidum* spores (GLSs) according to claim 16, wherein said pregnant female is a human.

18. The *Ganoderma lucidum* spores (GLSs) according to claim 16 or 17, wherein said gestational hypertension is caused by preeclampsia.

19. The *Ganoderma lucidum* spores (GLSs) according to claim 18, wherein said preeclampsia is caused by N-omega-nitro-L-arginine methyl ester.

20. A use of germination activated sporoderm-broken *Ganoderma lucidum* spores (GLSs) for the manufacture of a medicament for preventing or ameliorating increased expression of hippocampal hypoxia inducible factor (HIF-1a) in a mammalian embryo due to maternal gestational hypertension, wherein GLSs are administered to a female being pregnant with said mammalian embryo; wherein said pregnant female has gestational hypertension.

21. Germination activated sporoderm-broken *Ganoderma lucidum* spores (GLSs) for preventing or ameliorating increased expression of hippocampal vascular endothelial growth factor (VEGF) in a mammalian embryo.

22. The *Ganoderma lucidum* spores (GLSs) according to claim 21, wherein the increased expression of VEGF is due to maternal gestational hypertension, and wherein the GLSs is for administration to a female pregnant with said mammalian embryo; wherein said pregnant female has gestational hypertension.

23. The *Ganoderma lucidum* spores (GLSs) according to claim 22, wherein said pregnant female is a human.

24. The *Ganoderma lucidum* spores (GLSs) according to claim 22 or 23, wherein said gestational hypertension is caused by preeclampsia.

25. The *Ganoderma lucidum* spores (GLSs) according to claim 24, wherein said preeclampsia is caused by N-omega-nitro-L-arginine methyl ester.

26. A use of germination activated sporoderm-broken *Ganoderma lucidum* spores (GLSs) for the manufacture of a medicament for preventing or ameliorating increased expression of hippocampal hypoxia inducible factor (HIF-1a) in a mammalian embryo due to maternal gestational hypertension, wherein GLSs are administered to a female being pregnant with said mammalian embryo; wherein said pregnant female has gestational hypertension.
